(19) Europäisches Patentamt
European Patent Office
Office européen des brevets

(11) EP 0 575 785 B1

(12) **EUROPÄISCHE PATENTSCHRIFT**

(45) Veröffentlichungstag und Bekanntmachung des Hinweises auf die Patenterteilung:
**08.01.1997 Patentblatt 1997/02**

(51) Int Cl.[6]: **C07F 9/6568**, C07F 15/00, C07C 45/50

(21) Anmeldenummer: **93108829.8**

(22) Anmeldetag: **02.06.1993**

(54) **3,4-Dimethyl-2,5-6-tris(p-sulfonatophenyl)-1-phosphanorbornadien, Verfahren zu seiner Herstellung und Verfahren zur Hydroformylierung von olefinisch ungesättigten Verbindungen**

3,4-dimethyl-2,5,6-tris/p-sulfonatophenyl)-1-phosphanorbornadiene, process for its preparation and process for hydroformulation of olefinic unsaturated compounds

3,4-diméthyl-2,5,6-tris(p-sulfonaphényl)-1-phosphanorbornadiène, procédé pour sa préparation et procédé d'hydroformulation de composés à insaturation oléfinique

(84) Benannte Vertragsstaaten:
**AT BE DE ES FR GB IT NL SE**

(30) Priorität: **20.06.1992 DE 4220267**

(43) Veröffentlichungstag der Anmeldung:
**29.12.1993 Patentblatt 1993/52**

(73) Patentinhaber: **HOECHST AKTIENGESELLSCHAFT**
**65926 Frankfurt am Main (DE)**

(72) Erfinder:
- **Herrmann, Wolfgang A., Prof. Dr. Dipl.-Chem.**
  **W-8051 Giggenhausen (DE)**
- **Manetsberger, Rainer Dipl.-Chem.**
  **D-82407 Wielenbach (DE)**
- **Kohlpaintner, Christian, Dipl.-Chem.**
  **W-8209 Stephanskirchen (DE)**
- **Bahrmann, Helmut, Dr. Dipl.-Chem.**
  **W-4236 Hamminkeln 3 (DE)**

(56) Entgegenhaltungen:
**DE-A- 2 627 354** **FR-A- 2 588 197**

- **ANGEWANDTE CHEMIE. INTERNATIONAL EDITION Bd. 28, Nr. 4, 1989, WEINHEIM DE Seiten 500 - 501 DENIS NEIBECKER 'Phosphanorbornadienes as Ligands in the Transition Metal-Catalized Synthesis of Fine Chemicals'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 575 785 B1

## Beschreibung

Die Erfindung betrifft die chemische Verbindung 3,4-Dimethyl-2,5,6-tris(p-sulfonatophenyl)-1-phosphanorbornadien und ein Verfahren zu ihrer Herstellung sowie die Hydroformylierung olefinisch ungesättigter Verbindungen unter Verwendung von Katalysatoren, die die genannte Phosporverbindung enthalten.

Komplexverbindungen, die als Zentralatom ein Metall der 8. Gruppe des Periodensystems der Elemente und als Liganden P(III)-Verbindungen, Phosphine oder Phosphite und daneben gegebenenfalls noch weitere, zur Komplexbildung befähigte Gruppen enthalten, haben in jüngster Zeit zunehmend als Katalysatoren Bedeutung gewonnen. So erfolgt die technisch in großem Umfang ausgeübte Reaktion von Olefinen mit Synthesegas zu Aldehyden (Hydroformylierung) in Gegenwart von Katalysatorsystemen, die aus Kobalt und insbesondere Rhodium und Triphenylphosphin bestehen. Auch zur Umsetzung von Methanol mit Synthesegas zu höheren Alkoholen, insbesondere Ethanol und Propanol (Homologisierung) haben sich Katalysatoren auf Basis von Phosphin enthaltenden Komplexverbindungen bewährt. Zumeist liegen in den genannten Fällen die Liganden im Überschuß vor, so daß das Katalysatorsystem aus Komplexverbindung und freiem Ligand besteht. Entsprechend der Löslichkeit der Katalysatoren in organischen Medien erfolgen die Reaktionen in homogener Phase.

Statt in homogener Phase kann man die Umsetzung auch im heterogenen Reaktionssystem durchführen. Vorteil dieser Verfahrensvariante ist die einfache und schonende Trennung des Katalysators, der in Wasser gelöst vorliegt, von dem in Wasser nicht löslichen Reaktionsprodukt. Nach diesem Prinzip arbeitet z.B. das in der DE-C2 27 00 904 beschriebene Verfahren zur Anlagerung von Cyanwasserstoff an eine ungesättigte organische Verbindung mit mindestens einer ethylenischen Doppelbindung. Für die Herstellung von Aldehyden durch Reaktion von Olefinen mit Kohlenmonoxid und Wasserstoff setzt man nach dem Prozeß der DE-C2 26 27 354 Rhodium in metallischer Form oder in Form seiner Verbindungen zusammen mit einem wasserlöslichen Phosphin, z.B. dem Alkalisalz der Triphenylphosphintrisulfonsäure (im folgenden "TPPTS" genannt) als Katalysator ein.

Die bekannten Zweiphasen-Verfahren haben sich in technischem Maßstab sehr gut bewährt. Dennoch bemüht man sich um eine weitere Vervollkommnung des Prozesses. So versucht man, die Aktivität der Katalysatoren durch Modifizieren der Komplexliganden zu erhöhen und ihre Wirksamkeit zu verlängern, um den spezifischen Katalysatorbedarf - sowohl Rhodium als auch Ligand - und damit die Produktionskosten, weiter zu senken. Wirtschaftliche Gründe sind auch dafür maßgebend, auf eine deutliche Verminderung des Phosphin/Rhodium-Verhältnisses hinzuarbeiten. Darüber hinaus strebt man an, angepaßte Ligandensysteme zu entwickeln, die individuelle Probleme lösen. Als Beispiel sei die weitere Verbesserung der Selektivität hinsichtlich der Bildung unverzweigter Aldehyde genannt. Aber auch das entgegengesetzte Ziel, der Aufbau verzweigtkettiger Produkte, kann im Hinblick auf enantioselektive Synthesen von Interesse sein. Hierbei ist zu berücksichtigen, daß jährlich mehrere Millionen Tonnen Aus FR-A-2 588 197 ist 3,4-Dimethyl-2,5,6-triphenyl-1-phosphanorbornadien bekannt, das gemäß Angew. Chem. Int. Ed. Bd. 28, Nr. 4, 1989, 500 - 501, als Ligand für Katalysatorsysteme in Hydroformylierungsreaktionen in homogener Phase geeignet ist.

Hydroformylierungsprodukte hergestellt werden, so daß bereits eine geringe Änderung der Selektivität in die eine oder die andere Richtung wirtschaftlich bedeutsame Konsequenzen hat.

Gegenstand der Erfindung ist die chemische Verbindung 3,4-Dimethyl-2,5,6-tris(p-sulfonatophenyl)-1-phosphanorbornadien.

Ein weiterer Gegenstand der Erfindung ist ein Verfahren zur Herstellung von 3,4-Dimethyl-2,5,6-tris(p-sulfonatophenyl)-l-phosphanorbornadien. Es ist dadurch gekennzeichnet, daß man 3,4-Dimethyl-2,5,6-triphenyl-1-phosphanorbornadien bei Temperaturen von 0 bis 20°C mit einer Lösung von Schwefeltrioxid in Schwefelsäure umsetzt, unter Rühren allmählich auf Raumtemperatur einstellt und bei dieser Temperatur weitere 20 bis 30 min rührt, das Reaktionsgemisch unter Aufrechterhaltung einer Temperatur von 0 bis 50°C, insbesondere 0 bis 20°C mit Wasser verdünnt oder auf Eis gibt und anschließend aufarbeitet.

Weiterhin betrifft die Erfindung ein Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 30 MPa in Gegenwart in Wasser gelöster, Phosphine in komplexer Bindung enthaltender Rhodiumverbindungen als Katalysatoren. Es ist dadurch gekennzeichnet, daß als Phosphin 3,4-Dimethyl-2,5,6-tris(p-sulfonatophenyl)-1-phosphanorbornadien eingesetzt wird.

Überraschenderweise wurde gefunden, daß sich die neue Phosphorverbindung (im folgenden "NORBOS" genannt) hervorragend als Bestandteil wasserlöslicher Katalysatorsysteme, insbesondere solcher, die für Carbonylierungsreaktionen eingesetzt werden, eignet. NORBOS zeichnet sich durch hohe Oxidationsbeständigkeit und besondere Stabilität der Kohlenstoff-Phosphor-Bindungen aus. In komplexer Bindung mit Rhodium ergibt es einen vorzüglichen Hydroformylierungskatalysator, der die Umsetzung olefinisch ungesättigter Verbindungen zu Aldehyden mit hoher Aktivität A

$$A = \frac{\text{mol Aldehyd}}{\text{mol Rh. min}}$$

und Produktivität P

$$P = \frac{\text{g Aldehyd}}{\text{ml Katalysatorlösung . h}}$$

ermöglicht.

Die zur Herstellung von NORBOS benötigte Ausgangsverbindung 3,4-Dimethyl-2,5,6-triphenyl-1-phosphanorbornadien kann man nach dem Stand der Technik in guter Ausbeute in einer zweistufigen Synthese erhalten. Im ersten Verfahrensschritt wird Phenyldichlorphosphin oder Phenyldibromphosphin oder ein Gemisch der beiden Halogenphosphine mit Dimethylbutadien zum 1-Halo-2,5-dihydrophospholiumsalz umgesetzt, das man mit 2-Methylpyridin zum 3,4-Dimethyl-1-phenylphosphol dehydrohalogeniert. Die Reaktion des Phosphols mit Diphenylacetylen im zweiten Verfahrensschritt ergibt dann 3,4-Dimethyl-2,5,6-triphenyl-1-phosphanorbornadien.

Zur Sulfonierung kann das Phosphanorbornadien ohne vorherige Reinigung eingesetzt werden. Als Sulfonierungsmittel verwendet man erfindungsgemäß Oleum, d.h. eine Lösung von $SO_3$ in Schwefelsäure. Zweckmäßig beträgt die $SO_3$-Konzentration in dieser Lösung 20 bis 65 Gew.-%, bezogen auf die Lösung. Um die Reaktion unter schonenden Bedingungen durchzuführen, gibt man die Phosphorverbindung anteilsweise unter Aufrechterhaltung einer Temperatur von 0 bis 20, vorzugsweise von 0 bis 5°C in das $SO_3$-Reagenz, stellt unter Rühren allmählich auf Raumtemperatur ein und läßt in demselben Temperaturbereich 20 bis 30 min nachreagieren. Das Reaktionsgemisch wird darauf vorzugsweise jeweils unter Aufrechterhaltung einer Temperatur von 0 bis 50°C, insbesondere 0 bis 20°C mit Wasser oder besser mit Eis verdünnt und anschließend neutralisiert, vorzugsweise mit Alkalicarbonat oder Alkalihydroxid. Man engt die Lösung ein, filtriert von abgeschiedenem Alkalisulfat ab und trägt das Filtrat in Methanol ein. Falls erforderlich, kann die Reinigungsoperation, Einengen, Filtrieren und Eintragen in Methanol, wiederholt werden. Schließlich gewinnt man die sulfonierte Verbindung durch Eindampfen der Methanollösung zur Trockne. Das Rohprodukt kann unmittelbar als Bestandteil von Katalysatorsystemen verwendet werden. In reiner Form, so wie sie zur stofflichen Charakterisierung erforderlich ist, erhält man die neue Verbindung aus dem Rohprodukt z.B. durch Gelpermeationschromatographie.

Trinatrium-NORBOS ist ein gelblich weißes Pulver, das sich sehr leicht in Wasser löst. Aus dem Natriumsalz, wie auch aus anderen Alkalisalzen, können z.B. durch Ionenaustausch, die freie Säure und auch die Salze anderer Metalle hergestellt werden.

NORBOS bildet mit verschiedenen Metallen Komplexverbindungen, unter denen insbesondere die mit Rhodium Bedeutung als Katalysatoren für die Hydroformylierung olefinisch ungesättigter Verbindungen haben. Zweckmäßig werden hierbei Rhodium und Phosphorverbindung (in Form des Trinatriumsalzes oder eines anderen wasserlöslichen Salzes von NORBOS) nicht in stöchiometrischem Verhältnis, also entsprechend der chemischen Zusammensetzung der ursprünglich eingesetzten oder sich unter den Bedingungen der Hydroformylierungsreaktion bildenden Rhodium-Komplexverbindung verwendet. Vorteilhafter ist es, mit einem NORBOS-Überschuß zu arbeiten. Dabei kann man das Verhältnis von Rhodium und NORBOS in weiten Grenzen variieren und je mol Rhodium etwa 1 bis 15 mol NORBOS anwenden. Bevorzugt wird ein Verhältnis von ein mol Rhodium zu 3 bis 15 und insbesondere 8 bis 13 mol NORBOS.

Rhodium gelangt als Metall oder als Verbindung zum Einsatz. Metallisch verwendet man es entweder in Form feinverteilter Partikel oder in dünner Schicht auf einem Träger wie Aktivkohle, Calciumcarbonat, Aluminiumsilikat, Tonerde, niedergeschlagen. Als Rhodiumverbindungen kommen solche Substanzen in Betracht, die wasserlöslich sind oder unter den Reaktionsbedingungen wasserlöslich werden. Geeignet sind die verschiedenen Rhodiumoxide, die Salze anorganischer Wasserstoff- und Sauerstoffsäuren, sowie die Salze aliphatischer Mono- und Polycarbonsäuren. Beispiele für Rhodiumsalze sind Rhodiumnitrat, Rhodiumsulfat, Rhodiumacetat, Rhodium-2-ethylhexanoat, Rhodiummalonat. Rhodiumhalogenverbindungen kommen wegen des korrosiven Verhaltens der Halogenidionen dagegen weniger in Betracht. Weiterhin können Rhodiumcarbonylverbindungen wie $Rh_3(CO)_{12}$ oder $Rh_6(CO)_{16}$ oder Komplexsalze des Rhodiums, z.B. Cyclooctadienylrhodiumverbindungen eingesetzt werden. Bevorzugt werden Rhodiumoxid und insbesondere Rhodiumacetat und Rhodium-2-ethylhexanoat. Es ist anzunehmen, daß sich in Gegenwart von Wassergas unter den Bedingungen der Hydroformylierungsreaktion wasserlösliche Rhodium-Komplexverbindungen bilden, die Kohlenmonoxid und NORBOS als Liganden enthalten. Sie ergeben zusammen mit dem im Wasser gelösten überschüssigen NORBOS das Katalysatorsystem.

Die Katalysatorlösung wird aus den Komponenten entweder im Hydroformylierungsreaktor oder zuvor in einer separaten Vorrichtung hergestellt und darauf dem Hydroformylierungsreaktor zugeleitet.

Die Konzentration des Rhodiums in der wäßrigen Katalysatorlösung beträgt 20 bis 1000 Gew.-ppm (bezogen auf die Lösung), vorzugsweise 100 bis 600 Gew.-ppm und insbesondere 200 bis 400 Gew.-ppm.

Die Umsetzung des Olefins mit Kohlenmonoxid und Wasserstoff erfolgt bei Drücken von etwa 0,1 bis etwa 30 MPa, vorzugsweise 1 bis 12 MPa und insbesondere 3 bis 7 MPa. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumenverhältnis von Kohlenmonoxid und Wasserstoff etwa 1 : 1 ist oder von diesem Wert in der einen oder in der anderen Richtung

nur wenig abweicht.

Die Reaktionstemperatur liegt zwischen etwa 20 und 150°C, bevorzugt werden 80 bis 140°C und insbesondere 100 bis 125°C.

Die Umsetzung der in flüssiger und gasförmiger Phase vorliegenden Reaktionspartner erfolgt in konventionellen Reaktoren. Der Ablauf der Umsetzung wird maßgeblich dadurch beeinflußt, daß die wäßrige Katalysatorlösung mit dem flüssigen oder gasförmigen, hydrophoben Olefin und dem Synthesegas gesättigt werden muß. Daher ist es erforderlich, eine möglichst große Berührungsfläche zwischen den Phasen zu erzeugen. Es hat sich bewährt, den flüssigen Reaktorinhalt - Katalysatorlösung, gegebenenfalls flüssiges Olefin und Reaktionsprodukt - intensiv zu rühren und die gasförmigen Reaktionspartner - Synthesegas und gegebenenfalls Olefin - über Verteilungsvorrichtungen der flüssigen Phase zuzuführen. Es hat sich weiterhin sehr bewährt, den Anteil der organischen Phase im Reaktionsgemisch gering zu halten. Überraschenderweise trägt die organische Phase zur Löslichkeit der Reaktanten in der wäßrigen Phase nicht bei und es wird vermieden, daß das Reaktionsprodukt unerwünschte Nebenreaktionen eingeht, die bei zunehmender Verweilzeit des Produktes im Reaktor nicht auszuschließen sind. Dementsprechend stellt man ein Volumverhältnis von wäßriger zu organischer Phase von 1 : 1 bis 100 : 1, vorzugsweise 10 : 1 bis 100 : 1 ein. Zu diesem Zweck kann man kontinuierlich einen entsprechenden Teil des Reaktionsgemisches aus dem Reaktor ausschleusen, wäßrige und organische Phase voneinander trennen und die wäßrige Phase in den Reaktor zurückführen. Die Umsetzung kann absatzweise oder, bevorzugt, kontinuierlich durchgeführt werden.

Das erfindungsgemäße Verfahren ist mit Erfolg auf die Umsetzung von Monoolefinen, nicht konjugierten Polyolefinen, cyclischen Olefinen und Derivaten dieser ungesättigten Verbindungen anwendbar. Die Olefine können geradkettig oder verzweigt, die Doppelbindungen end- oder innenständig sein. Beispiele für Olefine, die in dem neuen Prozeß verwendet werden können sind Ethylen, Propylen, Buten-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol. Derivate der genannten Olefinarten, die nach der beanspruchten Arbeitsweise hydroformyliert werden können sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat, Diethylmaleinat, Acrylnitril. Mit besonderem Erfolg wird das Verfahren zur Hydroformylierung von Olefinen und Olefinderivaten mit 2 bis 12 Kohlenstoffatomen eingesetzt.

Die folgenden Beispiele erläutern die Erfindung, ohne sie auf die näher beschriebenen Ausführungsformen zu beschränken.

1. <u>Herstellung von NORBOS</u>

(a) 3,4-Dimethyl-l-phenylphosphol (nach A. Breque et al., Synth., 1981, 12, 984

In einem 1000 ml Rührkolben werden unter Stickstoff 44,7 g (250 mmol) Phenyldibromphosphin mit 66,9 g (250 mmol) Phenyldichlorphosphin 30 min bei Raumtemperatur zusammen gerührt. Dann gibt man zu der homogenen Lösung unter Eiskühlung 42,0 g (511 mmol) Dimethylbutadien und rührt unter weiterer Kühlung 24 h. Die entstehende Suspension läßt man weitere 10 d bei Raumtemperatur stehen. Sie wandelt sich dabei vollständig in eine weiße, kristalline Masse und der Feststoff wird im Kolben in möglichst kleine Stücke zerteilt und mit 300 ml Hexan und 200 ml Dichlormethan überschichtet.

Durch Einblasen von Stickstoff über einen Zeitaum von 30 min wird die Suspension von überschüssigem Dimethylbutadien befreit. Unter starkem Rühren fügt man bei Raumtemperatur tropfenweise eine Lösung von 103 g (1094 mmol) 2-Methylpyridin in 100 ml Dichlormethan zu. Im Verlauf von 24 h entsteht eine Mischung, die sich in zwei flüssige Phasen trennen laßt. Nach der Hydrolyse mit 100 ml 3 N Salzsäure trennt man die organische Phase ab und wäscht dreimal mit 100 ml Wasser bis zur Neutralität. Man trocknet über Natriumsulfat, filtriert und dampft ein. Die zurückbleibende Flüssigkeit wird mit 300 ml Hexan extrahiert, der Extrakt filtriert und eingedampft. Das so erhaltene Produkt bedarf keiner weiteren Reinigung.

Ausbeute: 71,17 g (378 mmol, 74,15 % der Theorie).

$^{31}P$-NMR (161,8 MHz,$CD_2Cl_2$): = -1,3 ppm (s).

(b) 3,4-Dimethyl-2,5,6-triphenyl-1-phosphanorbornadien (nach F. Mathey et al., J.Chem.Soc. 1981, 103, 4595)

Eine Lösung von 27,94 g (158 mmol) Diphenylacetylen und 29,50 g (158 mmol) 3,4-Dimethyl-1-phenylphosphol in 100 ml Toluol wird 3 d unter Rückfluß gekocht. Dann dampft man das Reaktionsgemisch im Ölpumpenvakuum zur Trockene ein und wäscht den Rückstand dreimal mit je 70 ml Pentan. Das Produkt kann aus Methylenchlorid umkristallisiert werden.

Ausbeute: 44,94 g (123 mmol, 77,6 % der Theorie)

$^{31}P$-NMR (161,8 MHz,$CD_2Cl_2$): = -7,4 ppm (s).

(c) Trinatrium-NORBOS

10,7 g (29,19 mmol) 3,4-Dimethyl-2,5,6-triphenyl-1-phosphanorbornadien werden unter Eiskühlung im Verlauf von 10 min in kleinen Anteilen unter intensivem Rühren zu 60 ml Oleum (25 % $SO_3$) gegeben. Nach einer Reaktionszeit von 1 h gibt man den Ansatz vorsichtig auf Eis. Anschließend neutralisiert man mit 25 %iger Natronlauge und dampft etwa auf ein Fünftel des ursprünglichen Volumens ein. Ausfallendes Natriumsulfat wird abfiltriert und das Filtrat in 400 ml Methanol eingerührt. Erneut ausfallendes Natriumsulfat wird abermals abfiltriert. Das Filtrat wird bis auf wenige ml eingeengt und in das vierfache Volumen Methanol eingespritzt. Suspendierte Feststoffteilchen werden abfiltriert, das Filtrat zur Trockene eingedampft.

Das Rohprodukt kann direkt als Katalysatorkomponente verwendet werden. Zur stofflichen Charakterisierung ist eine Reinigung mit Hilfe der Gelpermeationschromatographie unumgänglich.

Ausbeute: 17,2 g (23,67 mmol, 81,8 % der Theorie)

Elementaranalyse ber. für $C_{26}H_{26}Na_3O_{12}PS_3$ (M=726.62): ber.: C: 42.98, H: 3.61, O: 26.42, P: 4.26, S: 13.24, erh.: C: 43.31, H: 3.67, O: 26.56, P: 4.45, S: 13.08.

$^{31}$P-NMR (161,8 MHz, $D_2O$): = 5,3 ppm (s).

$^{31}$P-{H}-NMR (161,8 MHz, $D_2O$): -4.9 (s).

$^{1}$H-$^{1}$H-COSY-NMR- (400 MHz, $D_2O$): 2.0 (ABX, $^{1}$H,$^{2}J_{AB}$ = 10.2 Hz, $^{2}J_{AP}$ = 9.8 Hz, C$\underline{H}_A$-P), 1.84 (ABX, $^{1}$H, $^{2}J_{AB}$ 10.2 Hz, $^{2}J_{BP}$ =10.3 Hz, CH -P), 1.04 (s, 3H, Me), 1,78 (s, 3H, Me), 7.75 (d, 2H, $^{2}J_{HH}$ = 8.1 Hz, $C_{17,19}$-$\underline{H}$), 7.76 (d, 2H, $^{2}J_{HH}$ = 7.9 Hz, $C_{9,11}$-$\underline{H}$), 7.51 (d,2H, $^{2}J_{HH}$ = 7.9Hz, $C_{23,25}$-$\underline{H}$), 6.94 (d, 2H, $^{2}J_{HH}$ = 8.1 Hz, $C_{16,20}$-$\underline{H}$), 7.10 (d, 2H, $^{2}J_{HH}$ = 7.95 Hz, $C_{22,26}$-$\underline{H}$), 7.24 (d, 2H, $^{2}J_{HH}$ = 7.9 Hz, $C_{8,12}$-$\underline{H}$).

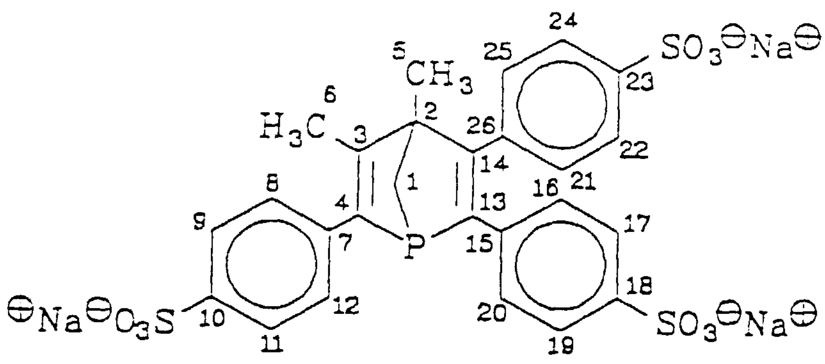

NORBOS–Na

2.) Hydroformylierung von Propen in Gegenwart von Rh/NORBOS als Katalysator

Einem mit Rührer ausgerüsteten 0,2 l Edelstahlautoklav werden Propylen und ein aus gleichen Volumenteilen bestehendes $CO/H_2$-Gemisch in einer solchen Menge zugeleitet, daß je Stunde 10 Nl Abgas aus dem Reaktor entnommen werden können. Gleichzeitig werden je Stunde 325 ml wäßrige Katalysatorlösung im Kreis durch den Reaktor geführt. Der Katalysator besteht aus 45 mg (0,44 mmol) Rhodium (als Acetat) und 5,89 mmol P(III) (in Form von Trinatrium-NORBOS), die in entgastem und mit Stickstoff gesättigtem Wasser zu 325 ml Lösung gelöst wurden. Das Molverhältnis von Phosphor zu Rhodium beträgt 13,4 : 1. Die Umsetzung der Reaktionspartner erfolgt bei einer Temperatur von 122°C und einem Druck von 5 MPa.

In der nachfolgenden Tabelle sind die Ergebnisse der Hydroformylierung von Propen bei unterschiedlichem Olefin-Einsatz zusammengestellt.

Tabelle: Hydroformylierung von Propen

| Versuch | Propen [g/h] | Produkt [g/h] | Aldehyd [%] | Selektivität [n/(n+iso)%] | Ges.-Alkohol [%] | C3-KW [%] | Andere [%] | Aktivität [n+iso] | Produktivität [n+iso] | Ausbeute [n+iso%] |
|---|---|---|---|---|---|---|---|---|---|---|
| 1 | 35,70 | 29,10 | 92,80 | 78,27 | 0,35 | 4,45 | 2,40 | 61,51 | 0,19 | 44,14 |
| 2 | 37,00 | 22,50 | 90,38 | 79,76 | 0,43 | 4,47 | 4,72 | 46,32 | 0,14 | 32,07 |
| 3 | 38,00 | 56,70 | 91,13 | 78,88 | 0,52 | 4,23 | 4,12 | 117,69 | 0,37 | 79,35 |
| 4 | 44,40 | 24,80 | 91,02 | 81,39 | 0,36 | 4,49 | 4,13 | 51,41 | 0,16 | 29,67 |
| 5 | 46,90 | 42,60 | 92,67 | 78,06 | 0,37 | 4,26 | 2,70 | 89,92 | 0,28 | 49,12 |
| 6 | 57,60 | 47,40 | 91,73 | 78,89 | 0,34 | 5,69 | 2,24 | 99,03 | 0,31 | 44,05 |

## Patentansprüche

1. Die chemische Verbindung 3,4-Dimethyl-2,5,6-tris(p-sulfonatophenyl)-1-phosphanorbornadien.

2. Verfahren zur Herstellung von 3,4-Dimethyl-2,5,6-tris( p-sulfonatophenyl)-l-phosphanorbornadien, dadurch gekennzeichnet, daß man 3,4-Dimethyl-2,5,6-triphenyl-1-phosphanorbornadien bei Temperaturen von 0 bis 20°C mit einer Lösung von Schwefeltrioxid in Schwefelsäure umsetzt, unter Rühren allmählich auf Raumtemperatur einstellt und bei dieser Temperatur weitere 20 bis 30 min rührt, das Reaktionsgemisch unter Aufrechterhaltung einer Temperatur von 0 bis 50°C, insbesondere 0 bis 20°C mit Wasser verdünnt oder auf Eis gibt und anschließend aufarbeitet.

3. Verfahren nach Anspruch 2, dadurch gekennzeichnet, daß die Konzentration des in der Schwefelsäure gelösten Schwefeltrioxids 20 bis 65 Gew.-%, bezogen auf die Lösung, beträgt.

4. Verfahren nach Anspruch 2 oder 3, dadurch gekennzeichnet, daß man das mit Wasser verdünnte Reaktionsgemisch unter Aufrechterhaltung einer Temperatur von 0 bis 50°C, insbesondere 0 bis 20°C mit einer wäßrigen Alkalicarbonat- oder Alkalihydroxid-Lösung neutralisiert, einengt, von abgeschiedenem Alkalisulfat abfiltriert, das Filtrat in Methanol einträgt und die Methanol-Lösung zur Trockene eindampft.

5. Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa in Gegenwart in Wasser gelöster, Phosphine in komplexer Bindung enthaltender Rhodiumverbindungen als Katalysatoren, dadurch gekennzeichnet, daß als Phosphin 3,4-Dimethyl-2,5,6-tris(p-sulfonatophenyl)-1-phosphanorbornadien eingesetzt wird.

6. Verfahren nach Anspruch 5, dadurch gekennzeichnet, daß je mol Rhodium 1 bis 15, vorzugsweise 3 bis 15 und insbesondere 8 bis 13 mol 3,4-Dimethyl-2,5,6-tris(p-sulfonatophenyl)-1-phosphanorbornadien eingesetzt werden.

7. Verfahren nach Anspruch 5 und 6, dadurch gekennzeichnet, daß die Rhodiumkonzentration in der wäßrigen Katalysatorlösung 20 bis 1000, vorzugsweise 100 bis 600 und insbesondere 200 bis 400 Gew.-ppm (bezogen auf die Lösung) beträgt.

8. Verfahren nach einem oder mehreren der Ansprüche 5 bis 7, dadurch gekennzeichnet, daß die Umsetzung bei 80 bis 140°C, insbesondere 100 bis 125°C erfolgt.

9. Verfahren nach einem oder mehreren der Ansprüche 5 bis 8, dadurch gekennzeichnet, daß die Umsetzung bei Drücken von 1 bis 12, vorzugsweise 3 bis 7 MPa erfolgt.

10. Verfahren nach einem oder mehreren der Ansprüche 5 bis 9, dadurch gekennzeichnet, daß Olefine oder Olefinderivate mit 2 bis 12 Kohlenstoffatomen umgesetzt werden.

## Claims

1. The chemical compound 3,4-dimethyl-2,5,6-tris(p-sulfonatophenyl)-1-phosphanorbornadiene.

2. A process for the preparation of 3,4-dimethyl-2,5,6-tris(p-sulfonatophenyl)-1-phosphanorbornadiene, which comprises reacting 3,4-dimethyl-2,5,6-triphenyl-1-phosphanorbornadiene with a solution of sulfur trioxide in sulfuric acid at temperatures of 0 to 20 °C, gradually bringing this to room temperature with stirring and stirring the mixture at this temperature for a further 20 to 30 min, keeping a temperature of 0 to 50 °C, in particular 0 to 20 °C, diluting the reaction mixture with water or adding it to ice and then working up the mixture.

3. The process as claimed in claim 2, wherein the concentration of the sulphur trioxide dissolved in the sulfuric acid is 20 to 65 % by weight, based on the solution.

4. The process as claimed in claim 2 or 3, wherein the reaction mixture diluted with water keeping a temperature of 0 to 50 °C, in particular 0 to 20 °C, is neutralized using an aqueous alkali metal carbonate solution or an alkali metal hydroxide solution, concentrated, filtered off from precipitated alkali metal sulfate, the filtrate is introduced

into methanol and the methanol solution is evaporated to dryness.

**5.** A process for the preparation of aldehydes by reaction of monoolefins, unconjugated polyolefins, cycloolefins or derivatives of these compound classes with carbon monoxide and hydrogen at temperatures of 20 to 150 °C and pressures of 0.1 to 20 MPa in the presence of rhodium compounds dissolved in water and containing complexed phosphines as catalysts, which comprises using 3,4-dimethyl-2,5,6-trio(p-sulfonatophenyl)-1-phosphanorbornadiene as phosphine.

**6.** The process as claimed in claim 5, wherein, per mole of rhodium, 1 to 15, preferably 3 to 15 and, in particular, 8 to 13 mol of 3,4-dimethyl-2,5,6-tris(p-sulfonatophenyl)-1-phosphanorbornadiene are used.

**7.** The process as claimed in claim 5 and 6, wherein the rhodium concentration in the aqueous catalyst solution is 20 to 1000, preferably 100 to 600, and, in particular, 200 to 400, ppm by weight (based on the solution).

**8.** The process as claimed in one or more of claims 5 to 7, wherein the reaction is carried out at 80 to 140 °C, in particular 100 to 125 °C.

**9.** The process as claimed in one or more of claims 5 to 8, wherein the reaction is carried out at pressures of 1 to 12, preferably 3 to 7, MPa.

**10.** The process as claimed in one or more of claims 5 to 9, wherein olefins or olefin derivatives having 2 to 12 carbon atoms are reacted.

## Revendications

**1.** Le composé chimique 3,4-diméthyl-2,5,6-tris(p-sulfonatophényl)-1-phosphanorbomadiène.

**2.** Procédé de préparation du 3,4-diméthyl-2,5,6-tris(p-sulfonatophényl)-1-phosphanorbornadiène, caractérisé en ce que l'on fait réagir le 3,4-diméthyl-2,5,6-triphényl-1-phosphanorbornadiène à des températures de 0 à 20°C avec une solution de trioxyde de soufre dans l'acide sulfurique, on règle peu à peu sous agitation à la température ambiante et on agite pendant encore 20 à 30 minutes à cette température, on dilue le mélange de réaction par l'eau ou on le jette sur glace en maintenant à une température de 0 à 50°C, plus spécialement de 0 à 20°C, et on procède à l'isolement.

**3.** Procédé selon la revendication 2, caractérisé en ce que la concentration du trioxyde de soufre en solution dans l'acide sulfurique est de 20 à 65 % du poids de la solution.

**4.** Procédé selon la revendication 2 ou 3, caractérisé en ce que le mélange de réaction dilué par l'eau est neutralisé à une température de 0 à 50°C, plus spécialement de 0 à 20°C, par une solution aqueuse de carbonate alcalin ou d'hydroxyde alcalin, soumis à concentration, débarrassé par filtration du sulfate alcalin qui a précipité, après quoi on coule le filtrat dans le méthanol et on évapore la solution méthanolique à sec.

**5.** Procédé de préparation d'aldéhydes par réaction de monooléfines, de polyoléfines non-conjuguées, de cyclooléfines ou de dérivés de ces classes de composés avec l'oxyde de carbone et l'hydrogène à des températures de 20 à 150°C et des pressions de 0,1 à 20 MPa en présence de catalyseurs consistant en composés du rhodium contenant des phosphines en liaison complexe et qui sont dissous dans l'eau, caractérisé on ce que la phosphine utilisée est le 3,4-diméthyl-2,5,6-tris(p-sulfonatophényl)-1-phosphanorbornadiène.

**6.** Procédé selon la revendication 5, caractérisé en ce que l'on utilise de 1 à 15, de préférence de 3 à 15 et plus spécialement de 8 à 13 mol de 3,4-diméthyl-2,5,6-tris(p-sulfonatophényl)-1-phosphanorbornadiène par mol de rhodium.

**7.** Procédé selon les revendications 5 et 6, caractérisé en ce que la concentration en rhodium de la solution aqueuse de catalyseur est de 20 à 1000, de préférence de 100 à 600 et plus spécialement de 200 à 400 ppm en poids par rapport à la solution.

**8.** Procédé selon une ou plusieurs des revendications 5 à 7, caractérisé en ce que la réaction est réalisée à une

température allant de 80 à 140°C, plus spécialement de 100 à 125°C.

**9.** Procédé selon une ou plusieurs des revendications 5 à 8, caractérisé en ce que la réaction est réalisée à des pressions de 1 à 12, de préférence de 3 à 7 MPa.

**10.** Procédé selon une ou plusieurs des revendications 5 à 9, caractérisé en ce que l'on convertit des oléfines ou dérivés d'oléfines contenant 2 à 12 atomes de carbone.